(19) Europäisches Patentamt
European Patent Office
Office européen des brevets

(11) **EP 4 491 097 A1**

(12) **EUROPEAN PATENT APPLICATION**
published in accordance with Art. 153(4) EPC

(43) Date of publication:
**15.01.2025 Bulletin 2025/03**

(21) Application number: **23766782.9**

(22) Date of filing: **06.03.2023**

(51) International Patent Classification (IPC):
***A61B 3/028*** (2006.01)

(52) Cooperative Patent Classification (CPC):
**A61B 3/028**

(86) International application number:
**PCT/JP2023/008295**

(87) International publication number:
**WO 2023/171606 (14.09.2023 Gazette 2023/37)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **09.03.2022 JP 2022035759**

(71) Applicant: **Hoya Lens Thailand Ltd.
Pathumthani 12130 (TH)**

(72) Inventor: **IIZUKA Takashi
Tokyo 160-8347 (JP)**

(74) Representative: **Betten & Resch
Patent- und Rechtsanwälte PartGmbB
Maximiliansplatz 14
80333 München (DE)**

(54) **REFRACTION CHARACTERISTICS MEASURING DEVICE**

(57)    Provided is a refractive characteristic measurement device (1) that measures a refractive characteristic of an eye (25), the device including: a light emission unit (20); an opening member (10) that is positioned between the light emission unit and the eye and has a first opening (11) and a second opening (12) through which light emitted by the light emission unit is narrowed and passed; a first optical element (13) that is provided in the first opening and transmits light in a first wavelength band; and a second optical element (14) that is provided in the second opening and transmits light in the second wavelength band, in which the light emission unit includes a background portion (23) that emits white light, a first visual target portion (21) that emits first light (L1) that is a complementary color to light in the first wavelength band, and a second visual target portion (22) that emits second light (L2) that is a complementary color to light in the second wavelength band.

FIG.1

EP 4 491 097 A1

## Description

Technical Field

The present invention relates to a refractive characteristic measurement device that measures a refractive characteristic of an eye.

Background Art

When eyeglasses or contact lenses are prescribed, a refraction inspection for measuring a refractive characteristic of an eye has been performed. As the refraction inspection, there are known a subjective inspection in which an inspection subject themselves identifies how presented visual target and light are seen, and an objective inspection in which a light beam incident on an eyeball is observed from the outside.

Patent Literature 1 proposes a device and a method capable of easily performing a refractive inspection of an eye by a subjective inspection. The device and the method utilize the Scheiner principle and are to provide two openings through which light is narrowed and passed in a measurement disk disposed in front of the eye and to measure a refractive characteristic of the eye based on how first light and second light that passed through the two openings and reached a retina are viewed (positional relationship between two images).

Citation List

Patent Literature

Patent Literature 1: Japanese Unexamined Patent Application Publication No. 2020-103743

Summary of Invention

Technical Problem

In the refraction inspection device and the refraction inspection method of Patent Literature 1, the two openings are set to have different transmission characteristics such that the first light and the second light are transmitted through only one corresponding openings. To perform an accurate inspection, a position and an orientation of the eye with respect to the measurement disk needs to be set so that the first light and the second light transmitted through respective openings are incident on a pupil at appropriate positions and reach the retina. Since the setting is performed based on how visual field regions corresponding to the two openings are viewed, easy viewing of the visual field region by a subject is important to realize accurate setting.

In addition, to inspect the refractive characteristic of the eye with high accuracy, the subject needs to easily identify a positional relationship between an image by the first light and an image by the second light.

To satisfy the above requirements, an object of the present invention is to improve accuracy and efficiency of an inspection in a refractive inspection device that measures a refractive characteristic by causing first light and second light to be incident to an eye through two openings.

Solution to Problem

According to an aspect of the present invention, provided is a refractive characteristic measurement device that measures a refractive characteristic of an eye, the device including: a light emission unit; an opening member that is positioned between the light emission unit and the eye and has a first opening and a second opening through which light emitted by the light emission unit is narrowed and passed; a first optical element that is provided in the first opening, transmits light in a first wavelength band, and blocks transmission of light in a second wavelength band; and a second optical element that is provided in the second opening, transmits the light in the second wavelength band, and blocks transmission of the light in the first wavelength band, in which the light emission unit includes a background portion that emits white light, a first visual target portion that emits first light that is a complementary color to the light in the first wavelength band, and a second visual target portion that emits second light that is a complementary color to the light in the second wavelength band.

It is preferable that the light in the first wavelength band is green light, the light in the second wavelength band is red light, the first light emitted by the first visual target portion is magenta light, and the second light emitted by the second visual target portion is cyan light.

As an example, it is preferable that the background portion emits the white light by combining blue component light having a maximum intensity in a wavelength band of 440 nm to 475 nm, green component light having a maximum intensity in a wavelength band of 520 nm to 550 nm, and red component light having a maximum intensity in a wavelength band of 600 nm to 650 nm, the first visual target portion emits the first light by combining the blue component light and the red component light, and the second visual target portion emits the second light by combining the blue component light and the green component light.

It is preferable that the first optical element satisfies the following conditions (1) and (2).

$$(1) \quad T_{B1}/T_{G1} < 1/10$$

$$(2) \quad T_{R1}/T_{G1} < 1/10$$

$T_{G1}$: a transmittance of the first optical element at a wavelength corresponding to the maximum intensity of the green component in the second light.

$T_{B1}$: a transmittance of the first optical element at a wavelength corresponding to the maximum intensity of the blue component in the first light.

$T_{R1}$: a transmittance of the first optical element at a wavelength corresponding to the maximum intensity of the red component in the first light.

It is preferable that the second optical element satisfies the following conditions (3) and (4).

$$(3) \quad T_{B2}/T_{R2} \; < \; 1/10$$

$$(4) \quad T_{G2}/T_{R2} \; < \; 1/10$$

$T_{R2}$: a transmittance of the second optical element at a wavelength corresponding to the maximum intensity of the red component in the first light.

$T_{B2}$: a transmittance of the second optical element at a wavelength corresponding to the maximum intensity of the blue component in the second light.

$T_{G2}$: a transmittance of the second optical element at a wavelength corresponding to the maximum intensity of the green component in the second light.

It is preferable that the first opening and the second opening have circular shapes with the same diameter, and a first visual target image having a shape corresponding to the first visual target portion and a second visual target image having a shape corresponding to the second visual target portion are observed in an overlapping region where a first visual field region observed through the first opening and a second visual field region observed through the second opening overlap each other.

Advantageous Effects of Invention

According to the present invention, in a refractive inspection device that measures a refractive characteristic by causing first light and second light to be incident to an eye through two openings, a visual field region and an image observed by a subject are easily viewed, and accuracy and efficiency of the inspection are improved.

Brief Description of Drawings

Fig. 1 is a diagram illustrating a schematic configuration of a refractive characteristic measurement device.

Fig. 2 is a diagram illustrating a position of an eye with respect to the refractive characteristic measurement device.

Fig. 3 is a diagram illustrating visual field regions and visual target images observed by a subject during inspection by the refractive characteristic measurement device.

Fig. 4 is a diagram illustrating an example of how a first visual target image and a second visual target image according to a refractive characteristic of an eye are viewed.

Fig. 5 is a diagram illustrating visual field regions and visual target images observed by a subject during inspection by a refractive characteristic measurement device of a comparative example.

Fig. 6 is a graph showing relative luminosity of visible light.

Fig. 7 is a diagram illustrating visual field regions and visual target images observed by the subject when a background portion of a light emission device is changed to white from the comparative example of Fig. 5.

Fig. 8 is a diagram individually illustrating how the first visual field region and the second visual field region observed by the subject are viewed in a refractive characteristic measurement device of the present embodiment.

Fig. 9 is a diagram illustrating visual field regions and visual target images observed by a subject during inspection by the refractive characteristic measurement device of the present embodiment.

Description of Embodiments

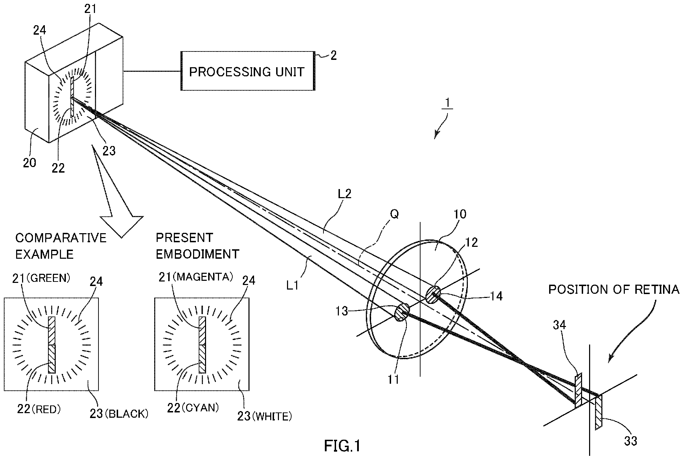

An outline of a refraction inspection (measurement of refractive characteristic) of an eye by a refractive characteristic measurement device 1 is described with reference to Figs. 1 to 4. The measurement of the refractive characteristic utilizes the Scheiner principle in which light passed through two different openings is refracted by a lens, intersects at a focal position to become one, and is separated into two at a position away from the focal position.

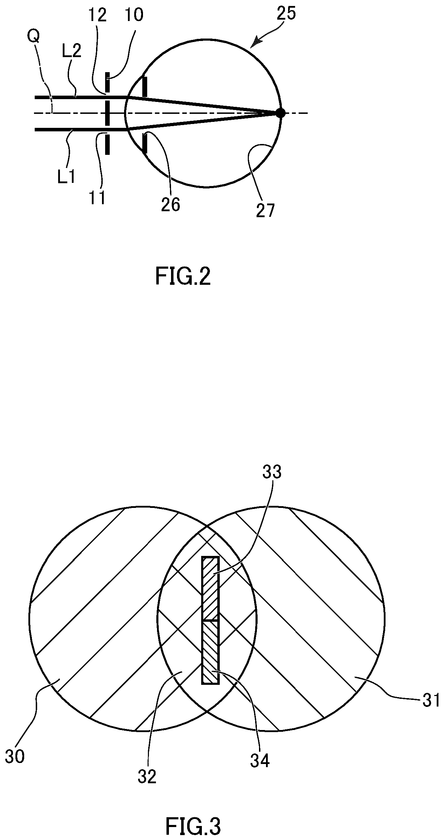

The refractive characteristic measurement device 1 includes a measurement disk (opening member) 10 and a light emission device (light emission unit) 20, and the measurement disk 10 is disposed between the light emission device 20 and an eye 25 (Fig. 2) of a subject.

The measurement disk 10 has a flat plate shape, and a first opening 11 and a second opening 12 are formed therein. The first opening 11 and the second opening 12 are circular openings that function as pinholes that narrow and pass light emitted by the light emission device 20. The first opening 11 and the second opening 12 have the same size (diameter). In addition, the sizes of the first opening 11 and the second opening 12 and the distance between the centers thereof are set to such an extent that the Scheiner principle acts. A direction in which the first opening 11 and the second opening 12 are aligned (a direction connecting centers of the first opening 11 and the second opening 12) is defined as an opening arrangement direction.

The light emission device 20 has a planar emission surface parallel to the measurement disk 10 and emits first light L1 and second light L2 from the emission surface toward the measurement disk 10 at the same distance. The light emission device 20 has two rectangular first and second visual target portions 21 and 22 on the emission surface, the first light L1 is emitted from the first visual target portion 21, and the second light L2 is emitted from the second visual target portion 22. Although details are described below, a color of the first light L1 is different from a color of the second light L2. In the emission surface of the light emission device 20, a region other than the first visual target portion 21 and the second visual target portion 22 is a background portion 23, and light can also be emitted from the background portion 23.

In the light emission device 20, relative positions of the first visual target portion 21 and the second visual target portion 22 can be changed along the opening arrangement direction of the measurement disk 10. In addition, angular positions of the first visual target portion 21 and the second visual target portion 22 can be changed about an axis perpendicular to the emission surface (an axis passing through a boundary between the first visual target portion 21 and the second visual target portion 22). Radial direction indicators 24 serving as indicators of the angular positions are formed on the background portion 23 around the first visual target portion 21 and the second visual target portion 22.

Various forms of light distribution by the light emission device 20 can be selected. For example, a transmissive surface light source (display) that causes regions of the first visual target portion 21, the second visual target portion 22, and the background portion 23 to respectively emit light in different colors can be used. Alternatively, the regions of the first visual target portion 21, the second visual target portion 22, and the background portion 23 may be set as light reflecting portions, and light reflected by the first visual target portion 21, the second visual target portion 22, and the background portion 23 may be distributed.

The first opening 11 is provided with a first optical element 13, and the second opening 12 is provided with a second optical element 14. Although details are described below, each of the first optical element 13 and the second optical element 14 has selective transmissivity (spectral characteristic) that transmits only light of different colors (wavelength bands). The first optical element 13 transmits light in a first wavelength band and blocks transmission of light in a second wavelength band. The second optical element 14 transmits the light in the second wavelength band and blocks transmission of the light in the first wavelength band. Using the selective transmissivity, a shape corresponding to the first visual target portion 21 is identified through the first opening 11, and a shape corresponding to the second visual target portion 22 is identified through the second opening 12.

As illustrated in Fig. 2, the measurement disk 10 is disposed between the light emission device 20 and the eye 25 of the subject such that a visual axis Q (virtual axis passing through the center of a pupil 26) of the eye 25 of the subject passes through the middle between the first opening 11 and the second opening 12. With such arrangement, the first light L1 passing through the first opening 11 and the second light L2 passing through the second opening 12 are incident on the pupil 26 of the eye 25 of the subject and reach a retina 27.

Under the above configuration and conditions, when the first light L1 is distributed from the first visual target portion 21, and the second light L2 is distributed from the second visual target portion 22, how the visual field region and the image observed by the subject are viewed is illustrated in Fig. 3. In the embodiment of the present invention and the comparative example described below, the visual field region and the image are colored, but here, the description of the difference in color is omitted, and an outline of the measurement of the refractive characteristic of the eye is described assuming that the subject can identify each visual field region and image.

As illustrated in Fig. 3, a substantially circular first visual field region 30 observed through the first opening 11 and a substantially circular second visual field region 31 observed through the second opening 12 overlap each other in a central overlapping region 32. A portion corresponding to the first visual target portion 21 is seen as a first visual target image 33, and a portion corresponding to the second visual target portion 22 is seen as a second visual target image 34.

When the eye 25 of the subject is located at an appropriate position with respect to the measurement disk 10 and the light emission device 20, the first visual target image 33 and the second visual target image 34 are viewed to be positioned on the overlapping region 32 as illustrated in Fig. 3.

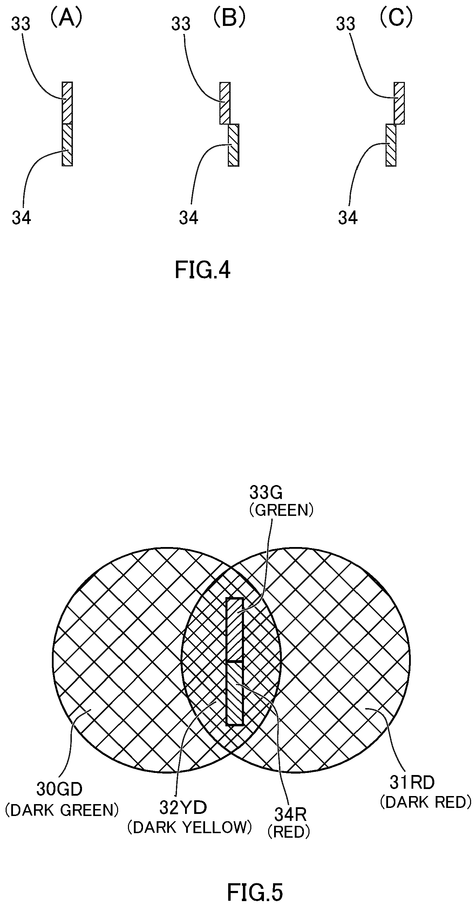

When the refractive characteristic of the eye 25 is appropriate, a position where the first light L1 passed through the first opening 11 reaches the retina 27 and a position where the second light L2 passed through the second opening 12 reaches the retina 27 match in the opening arrangement direction of the first opening 11 and the second opening 12. In this case, the subject views that the first visual target image 33 and the second visual target image 34 overlap in the opening arrangement direction. That is, when the first visual target portion 21 and the second visual target portion 22 each having a rectangular shape are linearly aligned on the light emission device 20 side, the first visual target image 33 and the second visual target image 34 each having a rectangular shape are viewed to be linearly aligned as illustrated in Fig. 4(A).

Meanwhile, when the refractive power of the eye 25 is larger than the appropriate refractive power of the eye, the first light L1 passed through the first opening 11 and the second light L2 passed through the second opening 12 intersect before reaching the retina 27. Conversely, when the refractive power of the eye 25 is smaller than the appropriate refractive power of the eye, the first light L1 passed through the first opening 11 and the second light L2 passed through the second opening 12 reach the retina 27 while not intersecting on the retina 27 and not intersecting before reaching the retina 27 (a position where the first light L1 and the second light L2 intersect with each other is behind the retina 27). Therefore, in these cases, the subject views that the first visual target image 33 and the second visual target image 34 deviate from each other in the opening arrangement direction. That is, when the first visual target portion 21 and the second visual target portion 22 each having a rectangular shape are linearly aligned on the light emission device 20 side, the first visual target image 33 and the second visual target image 34 each having a rectangular shape are viewed to be not linearly aligned. Fig. 4(B) illustrates an example of how the eye 25 views when the refractive power of the eye is larger than the appropriate refractive power, and Fig. 4(C) illustrates an example of how the eye views when the refractive power of the eye 25 is smaller than the appropriate refractive power.

Information on the refractive characteristic of the eye 25 can be obtained based on how the first visual target image 33 and the second visual target image 34 are viewed. However, it is difficult to quantitatively identify the deviation between the two images on the subject side and derive the refractive characteristic. Therefore, while a positional relationship between the first visual target portion 21 and the second visual target portion 22 is changed along the opening arrangement direction on the light emission device 20 side, a matching state in which the first visual target image 33 and the second visual target image 34 are viewed to overlap in the opening arrangement direction (the first visual target image 33 and the second visual target image 34 are viewed to be linearly aligned) is set, and the refractive power of the eye 25 is calculated from the positional relationship (amount of deviation or direction of deviation) between the first visual target portion 21 and the second visual target portion 22 in the matching state.

A processing unit 2 configuring the refractive characteristic measurement device 1 includes a computer or the like and controls position changes of the first visual target portion 21 and the second visual target portion 22 along the opening arrangement direction. For example, the positions of the first visual target portion 21 and the second visual target portion 22 are changed according to an input operation of the subject or a measurer with respect to an input device such as a keyboard or a touch panel. In addition, the processing unit 2 acquires distance information between the emission surface of the light emission device 20 and the measurement disk 10 from a distance sensor or the like. Then, the processing unit 2 calculates the refractive power of the eye 25 based on the positional deviation amount between the first visual target portion 21 and the second visual target portion 22 along the opening arrangement direction when the first visual target image 33 and the second visual target image 34 are in the above-described matching state on the retina 27. For the calculation of the refractive power of the eye 25, an equation described in Patent Literature 1 (Japanese Unexamined Patent Application Publication No. 2020-103743) described above and the like can be used.

According to the refractive characteristic measurement device 1 described above, the refractive power of the eye 25 can be accurately measured only by causing the subject to identify the matching state between the first visual target image 33 and the second visual target image 34. Since it is not required to perform complicated light emission control by the light emission device 20 or to remember the deviation amount between the first visual target image 33 and the second visual target image 34 by the subject or the measurer, the structure and control of the refractive characteristic measurement device 1 can be simplified, and time and effort for measurement are also reduced.

Since the refractive characteristic of the eye have orientation direction dependency, the above-described refractive characteristic measurement needs to be performed in a plurality of orientations in actual refractive inspection. Specifically, it is desirable that the opening arrangement directions of the first opening 11 and the second opening 12 are set to three or more directions of a horizontal direction, a vertical direction, and an intermediate orientation between the horizontal direction and the vertical direction, and measurement is performed in each direction.

In the refractive characteristic measurement device 1, the first opening 11 and the second opening 12 are made to have selective transmissivity of light so that only a shape corresponding to the first visual target portion 21 is observed through the first opening 11, and only a shape corresponding to the second visual target portion 22 is observed through the second opening 12. As a result, only two images of the first visual target image 33 and the second visual target image 34 are simultaneously viewed on the retina 27, and the subject can easily and accurately determine whether there is a positional deviation between the first visual target image 33 and the second visual target image 34.

To provide such selective transmissivity of light in the first opening 11 and the second opening 12, the first optical element 13 is provided in the first opening 11, and the second optical element 14 is provided in the second opening 12. The first optical element 13 and the second optical element 14 are optical filters that transmit light of different wavelength bands in the wavelength range of visible light.

Meanwhile, in the refractive characteristic measurement device 1 that performs the subjective inspection, by causing the first visual field region 30, the second visual field region 31, the overlapping region 32, the first visual target image 33, and the second visual target image 34 illustrated in Fig. 3 to be easily identified by the subject, accuracy and efficiency of the inspection can be improved. The present invention has been made by finding that visibility during observation by a subject is remarkably improved by causing the color of light emitted from each portion of the light emission device 20 and spectral characteristics in the first opening 11 (first optical element 13) and the second opening 12 (second optical element 14) of the measurement disk 10 to have a predetermined relationship, and details thereof are described below.

Note that Figs. 5, 7, and 9 illustrate a case where the two visual target images match with each other in the opening arrangement direction, but the case is an example, and the two visual target images may be viewed to be deviated in the opening arrangement direction as illustrated in Figs. 4(B) and 4(C) depending on the refractive characteristic of the eye 25.

First, a comparative example in which conditions are set different from the present invention is described. For example, in the subjective inspection of the eye in the related art, a method using a green or red observation index is known, and thus, the first light L1 emitted from the first visual target portion 21 of the light emission device 20 is set to green light, and the second light L2 emitted from the second visual target portion 22 is set to red light. The background portion 23 of the light emission device 20 is black. That is, in the comparative example, the light emission device 20 includes the first visual target portion 21 that emits green light, the second visual target portion 22 that emits red light, and the black background portion 23 (see a part of "comparative example" in Fig. 1).

As the first optical element 13, an optical filter that transmits green light and blocks transmission of red light is used. As the second optical element 14, an optical filter that transmits red light and blocks transmission of green light is used. That is, in the comparative example, the wavelength band of light transmitted by the first optical element 13 is substantially the same as the wavelength band of the first light L1 (green light) emitted from the first visual target portion 21, and the wavelength band of light transmitted by the second optical element 14 is substantially the same as the wavelength band of the second light L2 (red light) emitted from the second visual target portion 22.

By the function of the first optical element 13 and the second optical element 14, the first light L1 that is green light reaches the retina 27 of the eye 25 only through the first opening 11, and the second light L2 that is red light reaches the retina 27 of the eye 25 only through the second opening 12. Since the background portion 23 is black, only information of the first visual target portion 21 passes through the first opening 11, and only information of the second visual target portion 22 passes through the second opening 12.

How the visual field regions and the images are viewed by the subject here is described with reference to Fig. 5. A substantially circular first visual field region 30GD corresponding to the first opening 11 is obtained by observing the black background portion 23 through the first optical element 13 that transmits green light and is viewed in dark green (green with low brightness) due to the influence of ambient light. A substantially circular second visual field region 31RD corresponding to the second opening 12 is obtained by observing the black background portion 23 through the second optical element 14 that transmits red light and is viewed in dark red (red with low brightness) due to the influence of ambient light. An overlapping region 32YD is viewed in dark yellow (yellow with low brightness) due to the combination (additive color mixture) of dark green of the first visual field region 30GD and dark red of the second visual field region 31RD.

The green light emitted from the first visual target portion 21 is blocked from transmitting through the second opening 12 by the second optical element 14, transmits through only the first opening 11 including the first optical element 13, and is viewed as a green first visual target image 33G on the overlapping region 32. The red light emitted from the second visual target portion 22 is blocked from transmitting through the first opening 11 by the first optical element 13, transmits through only the second opening 12 including the second optical element 14, and is viewed as a red second visual target image 34R on the overlapping region 32.

When the eye 25 of the subject is at an appropriate position with respect to the measurement disk 10 and the light emission device 20, the first visual field region 30GD and the second visual field region 31RD are viewed to equally overlap each other, and the first visual target image 33G and the second visual target image 34R are positioned on the overlapping region 32YD, as illustrated in Fig. 5.

During the refraction inspection, it is required to set a position and an orientation of a face of the subject to achieve the above proper view. However, when the first visual field region 30GD and the second visual field region 31RD are dark green and dark red, respectively, with the black background of the background portion 23, there is a problem that the respective visual field regions are dark and thus hardly identified by the subject.

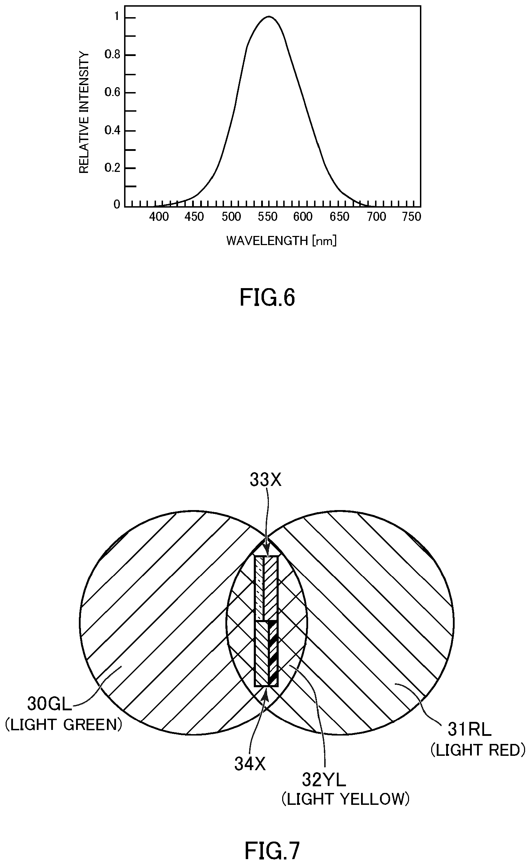

The relative luminosity of visible light is shown in Fig. 6. As can be understood from the graph of Fig. 6, red light (as an example, light in a wavelength band around 610 nm to 640 nm) has lower relative luminosity than green light (as an example, light in a wavelength band around 530 nm to 540 nm). Therefore, among the dark green first visual field region 30GD and the dark red second visual field region 31RD in Fig. 5, the second visual field region 31RD tends to be particularly hard to be viewed by the subject.

When the condition that the two visual target images are separately observed by the light passing through the first opening 11 and the light passing through the second opening 12 is satisfied, even if the two lights incident on the eye of the subject are not green light and red light, the refraction inspection can be performed. For example, the first visual target portion 21 may emit blue light (as an example, light in a wavelength band around 450 nm to 465 nm) and the blue light may pass through an optical filter as the first optical element 13. However, as can be understood from Fig. 6, blue light has lower relative luminosity than green light, the visual field region viewed through the first opening 11 tends to be hardly visible, and thus a problem as in the case of red light occurs.

Here, the inventor of the present application has focused on that, by emitting white light from the background portion 23 without setting the background portion 23 of the light emission device 20 as black, brightness of each of the visual field regions corresponding to the first opening 11 and the second opening 12 is increased, and thus the visual field regions are easily identified. How visual field regions and images are viewed by a subject when a change in which the background portion 23 emits white light is given to the above-described comparative example is described with reference to Fig. 7.

A substantially circular first visual field region 30GL corresponding to the first opening 11 is viewed light green (green with high brightness) due to a green component of the white light by observing the white light emitted from the background portion 23 through the first optical element 13 that transmits green light. A substantially circular second visual field region 31RL corresponding to the second opening 12 is viewed light red (red with high brightness) by observing the white light emitted from the background portion 23 through the second optical element 14 that transmits red light. An overlapping region 32YL is viewed in light yellow (yellow with high brightness) due to the combination (additive color mixture) of light green of the first visual field region 30GL and light red of the second visual field region 31RL. Therefore, as compared with the case where the background portion 23 is black (Fig. 5), the subject can easily identify the first visual field region 30GL, the second visual field region 31RL, and the overlapping region 32YL.

However, in the light emission device 20, when the first visual target portion 21 that emits green light and the second visual target portion 22 that emits red light are arranged on the background portion 23 that emits white light, the following problem occurs.

What is transmitted through the first opening 11 including the first optical element 13 includes not only the first light L1 (green light) emitted from the first visual target portion 21 but also a component of a predetermined wavelength band near green in the white light emitted from the background portion 23. Then, due to a refraction error between the light from the first visual target portion 21 and the light from the background portion 23, there is a possibility that a portion corresponding to the first visual target portion 21 does not become a single color of green but is viewed as a first visual target image 33X which is a double image in which portions having different colors are mixed.

What is transmitted through the second opening 12 including the second optical element 14 includes not only the second light L2 (red light) emitted from the second visual target portion 22 but also a component of a predetermined wavelength band near red in the white light emitted from the background portion 23. Then, due to a refraction error between the light from the second visual target portion 22 and the light from the background portion 23, there is a possibility that a portion corresponding to the second visual target portion 22 does not become a single color of red but is viewed as a second visual target image 34X which is a double image in which portions having different colors are mixed.

The first visual target image 33X and the second visual target image 34X which are viewed as double images have a problem that it is difficult for the subject to grasp the positional relationship in the opening arrangement direction. As described above, by only changing the background portion 23 to emit white light while maintaining the relationship in which the first visual target portion 21 and the second visual target portion 22 emit light of the same color as the light of the color transmitted by the first optical element 13 and the second optical element 14, the visual field regions (30GL, 31RL) corresponding to the first opening 11 and the second opening 12 are easily viewed, but there is a concern that it becomes difficult to identify the positional relationship between the first visual target image 33X and the second visual target image 34X respectively corresponding to the first visual target portion 21 and the second visual target portion 22.

As a result of research, the inventor of the present application has found that the above problem can be solved by causing the color of the light emitted from the first visual target portion 21 and the second visual target portion 22 in the light emission device 20 to be a complementary color to the color of the light in the first wavelength band transmitted by the first optical element 13 and a complementary color to the color of the light in the second wavelength band transmitted by the second optical element 14.

As a specific example, the first light L1 emitted from the first visual target portion 21 is set as magenta light which is a complementary color of green light (light in the first wavelength band) transmitted by the first optical element 13. In other words, the magenta color of the first light L1 is a color obtained by subtracting a component of green light (first wavelength band) from white light.

Also, the second light L2 emitted from the second visual target portion 22 is set as cyan light which is a complementary color of red light (light in the second wavelength band) transmitted by the second optical element 14. In other words, the cyan color of the second light L2 is a color obtained by subtracting a component of red light (second wavelength band) from white light.

That is, in the refractive characteristic measurement device 1 according to the embodiment to which the present invention is applied, the light emission device 20 includes the first visual target portion 21 that emits magenta light as the first light L1, the second visual target portion 22 that emits cyan light as the second light L2, and the background portion 23 that emits white light (see the part "present embodiment" in Fig. 1).

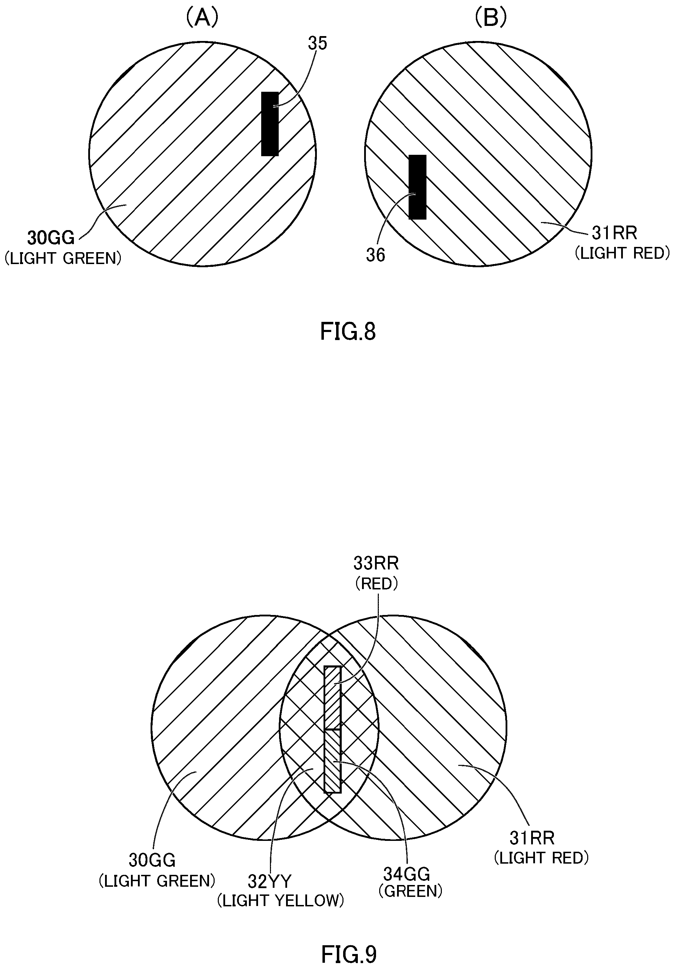

Fig. 8(A) illustrates how a first visual field region 30GG corresponding to the first opening 11 is viewed alone when the background portion 23 emits white light, the first visual target portion 21 emits the magenta first light L1, and the first optical element 13 is an optical filter that transmits only the wavelength band of green light. The first visual field region 30GG is viewed as in Fig. 8(A) when observation is performed only in the visual field of the first opening 11 by closing the second opening 12.

In Fig. 8(A), as for the entire first visual field region 30GG, similarly to the first visual field region 30GL in Fig. 7 described above, the background portion 23 that emits white light is observed through the first optical element 13, whereby the background portion is viewed in light green (green with high brightness) due to the green component of the white light. Since the magenta color is a color obtained by removing a green component from white (complementary color with respect to green), when the first visual target portion 21 emits the magenta first light L1, a figure presented by the first visual target portion 21 with respect to the transmission color (green) of the first optical element 13 becomes a shadow (black due to decoloring of complementary colors). As a result, a region of the first visual field region 30GG corresponding to the first visual target portion 21 becomes a first shadow portion 35 in which the shape of the first visual target portion 21 is not colored.

Fig. 8(B) illustrates how a second visual field region 31RR corresponding to the second opening 12 is viewed alone when the background portion 23 emits white light, the second visual target portion 22 emits the cyan second light L2, and the second optical element 14 is an optical filter that transmits only the wavelength band of red light. The second visual field region 31RR is viewed as in Fig. 8(B) when observation is performed only in the visual field of the second opening 12 by closing the first opening 11.

In Fig. 8(B), as for the entire second visual field region 31RR, similarly to the second visual field region 31RL in Fig. 7 described above, the background portion 23 that emits white light is observed through the second optical element 14, whereby the background portion is viewed in light red (red with high brightness) due to the red component of the white light. Since the cyan color is a color obtained by removing a red component from white (complementary color with respect to red), when the second visual target portion 22 emits the cyan second light L2, a figure presented by the second visual target portion 22 with respect to the transmission color (red) of the second optical element 14 becomes a shadow (black due to decoloring of complementary colors). As a result, a region of the second visual field region 31RR corresponding to the second visual target portion 22 becomes a second shadow portion 36 in which the shape of the second visual target portion 22 is not colored.

When the first visual target portion 21 that emits the magenta first light L1 is viewed in the visual field of the second opening 12, a red component and a magenta color in the white background in the background portion 23 cannot be distinguished by the function of the second optical element 14, and thus, the first visual target portion 21 is buried in the background portion 23, and the subject cannot recognize the shape of the first visual target portion 21.

When the second visual target portion 22 that emits the cyan second light L2 is viewed in the visual field of the first opening 11, a green component and a cyan color in the white background in the background portion 23 cannot be distinguished by the function of the first optical element 13, and thus, the second visual target portion 22 is buried in the background portion 23, and the subject cannot recognize the shape of the second visual target portion 22.

Therefore, the shape corresponding to the first visual target portion 21 is observed as the first shadow portion 35 only in the visual field of the first opening 11 (first visual field region 30GG). In addition, the shape corresponding to the second visual target portion 22 is observed as the second shadow portion 36 only in the visual field of the second opening 12 (second visual field region 31RR) .

The first visual field region 30GG of Fig. 8(A) and the second visual field region 31RR of Fig. 8(B) are combined, and how the visual field regions and the images observed by the subject are viewed in the refractive characteristic measurement device 1 of the present embodiment are illustrated in Fig. 9. When the eye 25 of the subject is at an appropriate position illustrated in Fig. 2 with respect to the measurement disk 10, the first visual field region 30GG and the second visual field region 31RR overlap with each other in a central overlapping region 32YY.

As described above, the first visual field region 30GG and the second visual field region 31RR are viewed brightly by observing the white light emitted by the background portion 23 through the first optical element 13 and the second optical element 14 and thus can be easily identified. In addition, similarly to the overlapping region 32YL of Fig. 7 described above, the overlapping region 32YY of Fig. 9 is viewed in light yellow due to the combination (additive color mixture) of light green of the first visual field region 30GG and light red of the second visual field region 31RR, and thus the subject can easily identify the overlapping region 32YY.

A portion corresponding to the first shadow portion 35 is positioned in the overlapping region 32YL. Then, the portion corresponding to the first shadow portion 35 is viewed as a rectangular first visual target image 33RR by the red component light (included in the second visual field region 31RR) transmitted through the second opening 12 (second optical element 14) in the white light emitted by the background portion 23. That is, a shape corresponding to the first visual target portion 21 that emits the magenta first light L1 in the light emission device 20 is observed as the red first visual target image 33RR.

A portion corresponding to the second shadow portion 36 is positioned in the overlapping region 32YL. Then, the portion corresponding to the second shadow portion 36 is viewed as a rectangular second visual target image 34GG by the green component light (included in the first visual field region 30GG) transmitted through the first opening 11 (first optical element 13) in the white light emitted by the background portion 23. That is, a shape corresponding to the second visual target portion 22 that emits cyan light in the light emission device 20 is observed as the green second visual target image 34GG.

As described above, by setting the color of the first light L1 and the color of the second light L2 such that a part corresponding to the first visual target portion 21 becomes the first shadow portion 35 in the first visual field region 30GG and a part corresponding to the second visual target portion 22 becomes the second shadow portion 36 in the second visual field region 31RR, when the first visual field region 30GG and the second visual field region 31RR overlap each other in the overlapping region 32YY, the part of the first shadow portion 35 becomes the first visual target image 33RR of a single color (red color), and the part of the second shadow portion 36 becomes the second visual target image 34GG of a single color (green color). As a result, the first visual target image 33RR and the second visual target image 34GG do not become images in which a plurality of colors are mixed such as the first visual target image 33X and the second visual target image 34X illustrated in Fig. 7 and thus are easily identified by the subject.

Therefore, all of the first visual field region 30GG, the second visual field region 31RR, the overlapping region 32YY, the first visual target image 33RR, and the second visual target image 34GG illustrated in Fig. 9 are observed to have high brightness and easily distinguishable colors, and the visibility by the subject is improved. More specifically, since the first visual field region 30GG, the second visual field region 31RR, and the overlapping region 32YY can be clearly identified, adjustment for positioning the first visual target image 33RR and the second visual target image 34GG on the overlapping region 32YY becomes easier in a measurement preparation stage, and appropriate alignment of the eye 25 with respect to the refractive characteristic measurement device 1 can be easily performed. In addition, since the first visual target image 33RR and the second visual target image 34GG are clearly seen as simple rectangular shapes of single colors of red and green, respectively, it becomes easier to determine a matching state between the first visual target image 33RR and the second visual target image 34GG in the opening arrangement direction. As a result, accuracy and efficiency of the inspection can be improved.

In the description of Fig. 9, the first visual field region 30GG is described as light green, the second visual field region 31RR is described as light red, the first visual target image 33RR is described as red, and the second visual target image 34GG is described as green, but such description does not mean that the first visual field region 30GG and the second visual field region 31RR are always viewed lighter than the first visual target image 33RR and the second visual target image 34GG. In comparison with dark green of the first visual field region 30GD and dark red of the second visual field region 30RD in the comparative example (Fig. 5), the first visual field region 30GG and the second visual field region 31RR are described as light green and light red in a sense that the first visual field region 30GG and the second visual field region 31RR are better in visibility.

In the first visual target image 33RR, since the red component included in the white light of the background portion 23 is placed on the first shadow portion 35, the first visual target image 33RR can be easily identified and clearly observed on the overlapping region 32YY. In the second visual target image 34GG, since the green component included in the white light of the background portion 23 is placed on the second shadow portion 36, the second visual target image 34GG can be easily identified and clearly observed on the overlapping region 32YY. Therefore, high visibility can be obtained not only for the first visual field region 30GG and the second visual field region 31RR but also for the first visual target image 33RR and the second visual target image 34GG.

As described above, when viewed in the visual field of the first opening 11 (Fig. 8(A)), when viewed in the visual field of the second opening 12 (Fig. 8(B)), and when viewed in the overlapping region of the two visual fields (overlapping region 32YY) (Fig. 9), the respective figures of the first visual target portion 21 and the second visual target portion 22 are viewed in a state of different colors. For example, when the eye 25 of the subject is not at an appropriate position with respect to the measurement disk 10, colors are not placed on the first shadow portion 35 and the second shadow portion 36, and thus the figures may not be viewed as the red first visual target image 33RR or the green second visual target image 34GG. Therefore, whether the eye 25 of the subject is at an appropriate position can be determined based on how the colors of the portions corresponding to the first visual target portion 21 and the second visual target portion 22 are viewed in addition to how the positional relationship of the first visual field region 30GG, the second visual field region 31RR, and the overlapping region 32YY are viewed.

The above effect is obtained by transmitting light in a green wavelength band and light in a red wavelength band by the first opening 11 (first optical element 13) and the second opening 12 (second optical element 14), respectively, emitting magenta light and cyan light which are complementary colors of the two colors by the first visual target portion 21 and the second visual target portion 22, and further emitting white light by the background portion 23. Since the present invention is characterized by the spectral characteristics imparted to the first optical element 13 and the second optical element 14 and the color setting of the light emitted from each region of the emission surface of the light emission device 20, it is excellent in that the present invention can be realized at low cost without requiring a complicated structure and control in the refractive characteristic measurement device 1.

As described above, since green has high relative luminosity, the use of green as the color of the first visual field region 30GG or the second visual target image 34GG improves the ease of identification of the portions. In addition, the use of red as the color of the second visual field region 31RR and the first visual target image 33RR facilitates visual distinguishment from the green first visual field region 30GG and the green second visual target image 34GG.

Next, a specific example of condition setting for realizing the above-described features in the refractive characteristic measurement device 1 is described. First, the light emission device 20 is described.

The light emitted from the background portion 23 of the light emission device 20 is preferably white light obtained by combining color components of following a1, a2, and a3.

a1: blue component having a maximum intensity at a wavelength of 440 mm to 475 nm.
a2: green component having a maximum intensity at a wavelength of 520 mm to 550 nm.
a3: red component having a maximum intensity at a wavelength of 600 mm to 650 nm.

The first light L1 emitted from the first visual target portion 21 of the light emission device 20 is preferably magenta light obtained by combining color components of following a1 and a3.

a1: blue component having a maximum intensity at a wavelength of 440 mm to 475 nm.
a3: red component having a maximum intensity at a wavelength of 600 mm to 650 nm.

The second light L2 emitted from the second visual target portion 22 of the light emission device 20 is preferably cyan light obtained by combining color components of following a1 and a2.

a1: blue component having a maximum intensity at a wavelength of 440 mm to 475 nm.
a2: green component having a maximum intensity at a wavelength of 520 mm to 550 nm.

When a transmissive surface light source capable of causing the regions of the first visual target portion 21, the second visual target portion 22, and the background portion 23 to respectively emit light in different colors is used as the light emission device 20, an LED backlight type or laser backlight type liquid crystal display is suitable in terms of high availability, ease of light emission control, and the like. In the three primary colors of light emitted from such a liquid crystal display, the center wavelength of the blue component is often in the range of 450 nm to 465 nm, the center wavelength of the green component is often in the range of 530 nm to 540 nm, and the center wavelength of the red component is often in the range of 610 nm to 640 nm. Then, considering variations in color development due to individual differences of products and temperature dependency, the values of a1, a2, and a3 are set with a margin of about ±10 nm in each range.

Next, suitable conditions for spectral characteristics in the first opening 11 and the second opening 12 are described.

The first optical element 13 provided in the first opening 11 is an optical filter that suppresses transmission of the first light L1 (magenta) and transmits a green component having a maximum intensity at a wavelength of 520 mm to 550 nm in the white light of the background portion 23 and preferably satisfies the following conditions (1) and (2).

$$(1)\ \ T_{B1}/T_{G1}\ <\ 1/10$$

$$(2)\ \ T_{R1}/T_{G1}\ <\ 1/10$$

$T_{G1}$ : transmittance at a wavelength $\lambda_{G2}$ corresponding to a maximum intensity of a green component (520 nm to 550 nm) in the second light L2 (cyan).
$T_{B1}$ : transmittance at a wavelength $\lambda_{B1}$ corresponding to a maximum intensity of a blue component (440 nm to 475 nm) in the first light L1 (magenta).
$T_{R1}$ : transmittance at a wavelength $\lambda_{R1}$ corresponding to a maximum intensity of a red component (600 nm to 650 nm) in the first light L1 (magenta).

By satisfying the condition (1), an effect of reducing crosstalk by suppressing the transmission of the blue component of the magenta first light L1 is obtained. By satisfying the condition (2), an effect of reducing crosstalk by suppressing the transmission of the red component of the magenta first light L1 is obtained. Therefore, by using the first optical element 13 that satisfies the conditions (1) and (2), the transmission of the first light L1 can be sufficiently suppressed, and the green component of the second light L2 can be sufficiently transmitted.

The second optical element 14 provided in the second opening 12 is an optical filter that suppresses transmission of the second light L2 (cyan) and transmits a red component having a maximum intensity at a wavelength of 600 mm to 650 nm in the white light of the background portion 23 and preferably satisfies the following conditions (3) and (4).

$$(3) \quad T_{B2}/T_{R2} < 1/10$$

$$(4) \quad T_{G2}/T_{R2} < 1/10$$

$T_{R2}$ : transmittance at the wavelength $\lambda_{R1}$ corresponding to the maximum intensity of the red component (600 nm to 650 nm) in the first light L1 (magenta).
$T_{B2}$ : transmittance at a wavelength $\lambda_{B2}$ corresponding to a maximum intensity of a blue component (440 nm to 475 nm) in the second light L2 (cyan).
$T_{G2}$: transmittance at a wavelength $\lambda_{G2}$ corresponding to a maximum intensity of a green component (520 nm to 550 nm) in the second light L2 (cyan).

By satisfying the condition (3), an effect of reducing crosstalk by suppressing the transmission of the blue component of the cyan second light L2 is obtained. By satisfying the condition (4), an effect of reducing crosstalk by suppressing the transmission of the green component of the cyan second light L2 is obtained. Therefore, by using the second optical element 14 that satisfies the conditions (3) and (4), the transmission of the second light L2 can be sufficiently suppressed, and the red component of the first light L1 can be sufficiently transmitted.
Next, examples of the light emission device 20, the first optical element 13, and the second optical element 14 configured by sample products are described.

<Example 1>

Light emission device 20 (liquid crystal display using LED backlight)

$\lambda_{B1}$ = 4 50 nm
$\lambda_{G2}$ = 540 nm
$\lambda_{R1}$ = 610 nm

First optical element 13 (bandpass filter having transmittance peak near wavelength of 530 nm)

$T_{B1}$ = 0.00
$T_{G1}$ = 0.54
$T_{R1}$ = 0.03
$T_{B1}/T_{G1}$ = 0.00
$T_{R1}/T_{G1} \approx 0.06$

<Example 2>

Light emission device 20 (liquid crystal display using LED backlight)

$\lambda_{B2}$ = 450 nm
$\lambda_{G2}$ = 540 nm
$\lambda_{R1}$ = 610 nm

Second optical element 14 (longpass filter having transmission limit wavelength near wavelength of 600 nm)

$T_{B2}$ = 0.00
$T_{G2}$ = 0.00
$T_{R2}$ = 0.65
$T_{B2}/T_{G2}$ = 0.00
$T_{R2}/T_{G2}$ = 0.00

<Example 3>

Light emission device 20 (liquid crystal display using laser backlight)

$\lambda_{B1}$ = 450 nm

$\lambda_{G2}$ = 540 nm

$\lambda_{R1}$ = 610 nm

First optical element 13 (bandpass filter having transmittance peak near wavelength of 535 nm)

$T_{B1}$ = 0.00
$T_{G1}$ = 0.63
$T_{R1}$ = 0.03
$T_{B1}/T_{G1}$ = 0.00
$T_{R1}/T_{G1} \approx 0.05$

<Example 4>

Light emission device 20 (liquid crystal display using laser backlight)

$\lambda_{B2}$ = 465 nm
$\lambda_{G2}$ = 530 nm
$\lambda_{R1}$ = 639 nm

Second optical element 14 (longpass filter having transmission limit wavelength near wavelength of 575 nm)

$T_{B2} = 0.00$
$T_{G2} = 0.00$
$T_{R2} = 0.88$
$T_{B2}/T_{G2} = 0.00$
$T_{R2}/T_{G2} = 0.00$

From the above, the first optical element 13 of each of Example 1 and Example 3 satisfies the condition (1) and the condition (2). Also, the second optical element 14 of each of Example 2 and Example 4 satisfies the condition (3) and the condition (4). The bandpass filter and the longpass filter having spectral characteristics exemplified in each example are widely distributed for color correction and color separation in optical devices and are excellent in availability.

Although the present invention is described above based on the illustrated embodiment, the present invention is not limited to the embodiment, and various modifications and changes can be made without departing from the gist of the invention.

In the above-described embodiment, the light in the first wavelength band transmitted by the first optical element 13 is green light, the light in the second wavelength band transmitted by the second optical element 14 is red light, the first visual target portion 21 emits the magenta first light L1 (complementary color of green), and the second visual target portion 22 emits the cyan second light L2 (complementary color of red). With such setting, the two visual field regions (first visual field region 30GG and second visual field region 31RR) and the two visual target images (first visual target image 33RR and second visual target image 34GG) observed by the subject through the first opening 11 and the second opening 12 become green having high relative luminosity and red having a relationship close to negative afterimage color with respect to green, and visibility by the subject can be improved.

However, it is also possible to change the color setting from the above-described embodiment with respect to the light in the wavelength band transmitted by each of the first optical element 13 and the second optical element 14, the first light L1 emitted by the first visual target portion 21, and the second light L2 emitted by the second visual target portion 22. In the present invention, given that the first optical element provided in the first opening transmits the light in the first wavelength band and blocks the transmission of the light in the second wavelength band, and the second optical element provided in the second opening transmits the light in the second wavelength band and blocks the transmission of the light in the first wavelength band, at least a condition that the first light emitted by the first visual target portion is a complementary color to the light in the first wavelength band and a condition that the second light emitted by the second visual target portion is a complementary color to the light in the second wavelength band needs to be satisfied. Colors having a relationship satisfying such conditions exist in addition to each color of the above-described embodiment, and it is possible to appropriately select the setting of each color based on factors such as visibility from the subject during inspection and productivity of the light emission unit.

The detailed configuration of the refractive characteristic measurement device may be different from that of the refractive characteristic measurement device 1 of the above-described embodiment. For example, as the shapes of the first opening 11 and the second opening 12 provided in the measurement disk 10, shapes other than a circle can be selected. In addition, the shapes of the first visual target portion 21 and the second visual target portion 22 may be other than a rectangle.

Industrial Applicability

By applying the present invention, it is possible to improve accuracy and efficiency of inspection in a refraction inspection device that measures a refractive characteristic of an eye.

This application is based on Japanese Patent Application No. 2022-035759 filed on March 9, 2022. All the contents are included herein.

Claims

1. A refractive characteristic measurement device that measures a refractive characteristic of an eye, the device comprising:

    a light emission unit;
    an opening member that is positioned between the light emission unit and the eye and has a first opening and a second opening through which light emitted by the light emission unit is narrowed and passed;
    a first optical element that is provided in the first opening, transmits light in a first wavelength band, and blocks transmission of light in a second wavelength band; and
    a second optical element that is provided in the second opening, transmits the light in the second wavelength band, and blocks transmission of the light in the first wavelength band, wherein the light emission unit includes a background portion that emits white light, a first visual target portion that emits first light that is a complementary color to the light in the first wavelength band, and a second visual target portion that emits second light that is a complementary color to the light in the second wavelength band.

2. The refractive characteristic measurement device according to claim 1, wherein the light in the first wavelength band is green light, the light in the second wavelength band is red light, the first light emitted by the first visual target portion is magenta light, and the second light emitted by the second visual target portion is cyan light.

3. The refractive characteristic measurement device according to claim 2, wherein

the background portion emits the white light by combining light of blue component having a maximum intensity in a wavelength band of 440 nm to 475 nm, light of green component having a maximum intensity in a wavelength band of 520 nm to 550 nm, and light of red component having a maximum intensity in a wavelength band of 600 nm to 650 nm,
the first visual target portion emits the first light by combining the light of blue component and the light of red component, and
the second visual target portion emits the second light by combining the light of blue component and the light of green component.

4. The refractive characteristic measurement device according to claim 3, wherein the first optical element satisfies the following conditions (1) and (2):

$$(1) \quad T_{B1}/T_{G1} < 1/10$$

$$(2) \quad T_{R1}/T_{G1} < 1/10$$

$T_{G1}$: a transmittance of the first optical element at a wavelength corresponding to the maximum intensity of the green component in the second light;
$T_{B1}$: a transmittance of the first optical element at a wavelength corresponding to the maximum intensity of the blue component in the first light; and
$T_{R1}$: a transmittance of the first optical element at a wavelength corresponding to the maximum intensity of the red component in the first light.

5. The refractive characteristic measurement device according to claim 3, wherein the second optical element satisfies the following conditions (3) and (4):

$$(3) \quad T_{B2}/T_{R2} < 1/10$$

$$(4) \quad T_{G2}/T_{R2} < 1/10$$

$T_{R2}$: a transmittance of the second optical element at a wavelength corresponding to the maximum intensity of the red component in the first light;
$T_{B2}$: a transmittance of the second optical element at a wavelength corresponding to the maximum intensity of the blue component in the second light; and
$T_{G2}$: a transmittance of the second optical element at a wavelength corresponding to the maximum intensity of the green component in the second light.

6. The refractive characteristic measurement device according to any one of claims 1 to 5, wherein

the first opening and the second opening have circular shapes with a same diameter, and
a first visual target image having a shape corresponding to the first visual target portion and a second visual target image having a shape corresponding to the second visual target portion are observed in an overlapping region where a first visual field region observed through the first opening and a second visual field region observed through the second opening overlap each other.

FIG.1

FIG.2

FIG.3

FIG.4

33G
(GREEN)

30GD
(DARK GREEN)

32YD
(DARK YELLOW)

34R
(RED)

31RD
(DARK RED)

FIG.5

FIG.6

FIG.7

(A)                                (B)

35

30GG                               31RR
(LIGHT GREEN)        36            (LIGHT RED)

FIG.8

33RR
(RED)

30GG                                                       31RR
(LIGHT GREEN)      32YY            34GG                     (LIGHT RED)
              (LIGHT YELLOW)    (GREEN)

FIG.9

## INTERNATIONAL SEARCH REPORT

| International application No. |
| --- |
| **PCT/JP2023/008295** |

**A. CLASSIFICATION OF SUBJECT MATTER**

*A61B 3/028*(2006.01)i
FI: A61B3/028

According to International Patent Classification (IPC) or to both national classification and IPC

**B. FIELDS SEARCHED**

Minimum documentation searched (classification system followed by classification symbols)

A61B3/028

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
| --- | --- | --- |
| A | JP 2020-103743 A (HOYA LENS THAILAND LTD) 09 July 2020 (2020-07-09) paragraphs [0022]-[0049], fig. 1-6 | 1-6 |
| A | US 2002/0140903 A1 (SCHACHAR, Ronald A.) 03 October 2002 (2002-10-03) paragraphs [0046]-[0075], fig. 1-13 | 1-6 |

☐ Further documents are listed in the continuation of Box C.    ☑ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| **29 March 2023** | **18 April 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (January 2015)

| | INTERNATIONAL SEARCH REPORT<br>Information on patent family members | | International application No.<br>**PCT/JP2023/008295** | |

| Patent document<br>cited in search report | Publication date<br>(day/month/year) | Patent family member(s) | Publication date<br>(day/month/year) |
|---|---|---|---|
| JP 2020-103743 A | 09 July 2020 | US 2022/0054005 A1<br>paragraphs [0055]-[0103], fig.<br>1-6<br>WO 2020/137533 A1<br>EP 3903663 A1<br>CN 113260298 A | |
| US 2002/0140903 A1 | 03 October 2002 | WO 2002/078530 A1 | |

Form PCT/ISA/210 (patent family annex) (January 2015)

**REFERENCES CITED IN THE DESCRIPTION**

**Patent documents cited in the description**

- JP 2020103743 A
- JP 2022035759 A